Europäisches Patentamt

(19) European Patent Office                    (11) Publication number:    **0 075 372**

Office européen des brevets                                          **B1**


(12)                    **EUROPEAN PATENT SPECIFICATION**


(45) Date of publication of patent specification: **07.08.85**    (51) Int. Cl.⁴: **C 07 D 201/08**

(21) Application number: **82201164.9**

(22) Date of filing: **21.09.82**

(54) **Process for the preparation of a lactam.**

(30) Priority: **22.09.81 NL 8104344**

(43) Date of publication of application:
**30.03.83 Bulletin 83/13**

(45) Publication of the grant of the patent:
**07.08.85 Bulletin 85/32**

(84) Designated Contracting States:
**AT BE CH DE FR GB IT LI NL SE**

(56) References cited:
**DE-A-1 545 738**
**FR-A-1 527 707**
**US-A-2 681 349**
**US-A-3 235 562**

(73) Proprietor: **STAMICARBON B.V.**
**Postbus 10**
**NL-6160 MC Geleen (NL)**


(72) Inventor: **van der Stoel, Roland Emile**
**Schout Boutenstraat 27**
**NL-6121 HC Buchten (NL)**
Inventor: **Bosma, Marcel Antoine Robert**
**Thorbeckestraat 26**
**NL-6162 XT Geleen (NL)**
Inventor: **Janssen, Petrus Hubert Joseph**
**Mauritspark 32**
**NL-6163 HN Geleen (NL)**
Inventor: **van de Moesdijk, Cornelis Gerardus**
**Maria**
**Drossaert Saldenstraat 7**
**NL-6181 ER Elsloo (L.) (NL)**


(74) Representative: **Hoogstraten, Willem Cornelis**
**Roeland et al**
**OCTROOIBUREAU DSM Postbus 9**
**NL-6160 MA Geleen (NL)**

Courier Press, Leamington Spa, England.

## Description

The invention relates to a process for the preparation of a lactam by reductive amination of an oxo-alkanecarboxylic compound in the presence of a hydrogenation catalyst.

According to the United States specification 3 235 562 such a process may very well be performed in the gas phase. This patent specification advises strongly against performing this process in the liquid phase in a solvent, because in that case, among other things, a lower lactam yield is obtained on account of undesired side reactions.

Such liquid phase processes in the presence of a solvent are as such known from e.g. US—A— 2 681 349, FR—A—1 527 707 and DE—A— 1 545 738. In these processes a very high $H_2$ pressure is applied and moreover rather long reaction times are required.

It has now been surprisingly found that the performance of this process in the liquid phase in the absence of a solvent produces a very good result.

The process according to the invention for the preparation of a lactam by reductive amination of an oxo-alkanecarboxylic compound in the presence of a hydrogenation catalyst is characterized in that an oxo-alkanecarboxylic acid ester with 2 or 3 carbon atoms between the carbon atom of the carbonyl group and the carbon atom of the carboxyl group is subjected to reductive amination in the liquid phase in the absence of a solvent.

Suitable starting esters are for example esters of 5-oxo-caproic acid, 4-methyl-5-oxocaproic acid and laevulinic acid. The ester can be formed as known in the art from the relative acid and an alcohol. When using the ester, an alcohol corresponding with the ester group is obtained as side product. Various ester groups can be used such as, for instance, an alkyl, cycloalkyl or benzyl group. Preference is given to a lower alkyl group such as a methyl, ethyl and isopropyl group.

The reaction according to the invention can be performed with hydrogenation catalysts known in the art, for instance catalysts containing a metal or compound of a metal from the 8th group or 1st subgroup of the periodic system of the elements according to Mendeleev. The chosen quantity of catalyst may vary, for instance a quantity of 0.01 g to 20 g catalyst, calculated as metal, per 100 g starting compound. As more catalyst is used, a shorter reaction time will suffice to achieve the same result. Preference is given to using, per 100 g starting compound, 0.1 g to 10 g catalyst, calculated as metal. If so desired, a promotor can be used as well. The catalysts can be applied on a carrier material such as, for instance, activated carbon, graphite, silica, magnesiumoxide and mixtures of two or more of these materials. Preference is given to using a paladium, platinum or nickel-containing hydrogenation catalyst. Particularly suitable are nickel-containing hydrogenation catalyst.

The process according to the invention can be performed at various temperatures, for instance a temperature of between 50 and 300°C. Preference is given to applying a temperature of between 100 and 250°C. The pressure is not critical, but must be chosen so as to make it possible for the relative reaction to take place in the liquid phase.

The process according to the invention can be performed while applying hydrogen having a partial hydrogen pressure of, for instance, 1—150 bar. A partial hydrogen pressure higher than 150 bar can be applied as well, but this does not result in an advantage. In addition to hydrogen, ammonia or a primary amine must be present in the reaction mixture for the formation of the lactam according to the invention. If a primary amine is applied, an N-substituted lactam is obtained with a substituent corresponding with the primary amine applied. Any primary amine which can be present in the liquid phase under the reaction conditions can be applied. The quantity of ammonia or primary amine can be varied, for instance from 1—10 moles per mole starting product to be converted. A quantity of ammonia or primary amine larger than 10 moles per mole starting product to be converted can be applied as well, but in doing so no advantage is achieved. Preference is given to using 1—3 moles ammonia or primary amine per mole starting product to be converted.

For the practical realization of the reductive amination according to the invention, the modes of realizing catalytic liquid phase reactions known per se are eligible, for instance the realization in a stirred reactor with catalyst suspended in the liquid phase or the mode of realization in which liquid starting product is passed over a fixed catalyst bed.

The lactams that can be prepared according to the invention are suitable for various uses, for instance in the preparation of plastics.

The invention is further elucidated in the following examples.

Example I

A stirred autoclave having a capacity of 0.5 l, provided with a storage tank, is filled with 21 g ammonia and 6.2 g Raney nickel. The storage tank contains 100.4 g methyl ester of 5-oxocaproic acid. The mixture in the autoclave is heated to 110°C. The oxo-ester from the storage tank is subsequently added to the mixture in the autoclave and simultaneously hydrogen (purity 99.9 %) is metered up to a total pressure of 50 bar. In consequence of the reaction that occurs the temperature rises in a short time to 173°C. This temperature is maintained for 30 minutes and by the addition of hydrogen the total pressure is kept at 50 bar for this period. After cooling, the contents of the autoclave are diluted with ethanol and analyzed by means of gas chromatography. From this analysis the conversion of the oxo-ester and the yield of product formed (6-methylpiperidone-2 and · 6-methyl-3,4-dihydro-pyridone-2) can be calculated.

The conversion of the oxo-ester is understood to mean the quantity of oxo-ester converted, expressed as a percentage of the quantity of oxo-ester added. The yield is understood to mean the quantity of the relative product than can theoretically be formed from the quantity of oxo-ester converted, expressed as a percentage of the quantity of oxo-ester converted.

The conversion is 100 %. The yield of 6-methylpiperidone-2 93.2 % and 6-methyl-3,4-dihydro-pyridone-2 3.3 %.

Example II

In the way described in Example I, 100.6 g methyl ester of 5-oxocaproic acid is added, at 143°C, to 19 g ammonia and 5.67 g platinum on carbon catalyst (5 % by wt. Pt). During this addition the temperature rises to 202°C. This temperature is maintained for 60 minutes at a total pressure of 50 bar. The conversion is 99.5 % and the yield of 6-methylpiperidone-2 80.8 %.

Example III

In the way described in Example I, 100.4 g methyl ester of 5-oxocaproic acid is added, at 154°C, to 12 g ammonia and 5.75 g palladium on carbon catalyst (10 % by wt. Pd). During this addition the temperature rises to 223°C. After this temperature has been maintained for 60 minutes at a total pressure of 50 bar, the conversion is 97.2 % and the yield of 6-methylpiperidone-2 88 %.

Example IV

In the way described in Example I, 102.2 g methyl ester of 5-oxocaproic acid is added, at 150°C, to 20 g ammonia and 5.08 g nickel on silica catalyst (60 % by wt. Ni). During the addition the temperature rises to 222°C. After this temperature has been maintained for 30 minutes at a total pressure of 50 bar, the conversion is 98.3 %, the yield of 6-methylpiperidone-2 94 % and of 6-methyl-3,4-dihydropyridone-2 2.9 %.

Example V

In the way described in Example I, 100 g methyl ester of 4-methyl-5-oxocaproic acid is added, at 150°C, to 19 g ammonia and 5.12 g nickel on silica catalyst (60 % wt. Ni). During this addition the temperature rises to 207°C. After this temperature has been maintained for 30 minutes at a total pressure of 50 bar, the conversion is 98 % and the yield of 5,6-dimethylpiperidone-2 85 %.

**Claims**

1. Process for the preparation of a lactam by reductive amination of an oxo-alkanecarboxylic compound in the presence of a hydrogenation catalyst, characterized in that an oxo-alkanecarboxylic acid ester with 2 or 3 carbon atoms between the carbon atom of the carbonyl group and the carbon atom of the carboxyl group, is subjected to reductive amination in the liquid phase in the absence of a solvent.

2. Process according to claim 1, characterized in that as an ester the methyl, ethyl or isopropyl ester is used.

3. Process according to claim 1 or 2, characterized in that per 100 g starting compound 0.1—10 g catalyst calculated as metal is used.

4. Process according to any one of claims 1—3, characterized in that a palladium, platinum or nickel-containing hydrogenation catalyst is used.

5. Process according to claim 4, characterized in that a nickel-containing hydrogenation catalyst is used.

6. Process according to any one of claims 1—5, characterized in that the reductive amination is performed at a temperature of 100—250°C.

7. Process according to any one of claims 1—6, characterized in that per mole starting product to be converted 1—3 moles ammonia or primary amine is used.

**Patentansprüche**

1. Verfahren zur Herstellung eines Lactams durch reduktive Aminierung einer Oxo-alkancarbonsäureverbindung in Gegenwart eines Hydrierungskatalysators, dadurch gekennzeichnet, daß ein Oxo-alkancarbonsäureester mit 2 oder 3 Kohlenstoffatomen zwischen dem Kohlenstoffatom der Carbonylgruppe und dem Kohlenstoffatom der Carboxylgruppe einer reduktiven Aminierung in der flüssigen Phase in Abwesenheit eines Lösungsmittels unterworfen wird.

2. Verfahren nach Anspruch 1, dadurch gekennzeichnet, daß als Ester der Methyl-, Äthyl- oder Isopropylester eingesetzt wird.

3. Verfahren nach Anspruch 1 oder 2, dadurch gekennzeichnet, daß je 100 g Ausgangsverbindung 0,1—10 g Katalysator, berechnet als Metall, verwendet wird.

4. Verfahren nach einem der Ansprüche 1—3, dadurch gekennzeichnet, daß ein Palladium, Platin oder Nickel enthaltender Hydrierungskatalysator verwendet wird.

5. Verfahren nach Anspruch 4, dadurch gekennzeichnet, daß ein Nickel enthaltender Hydrierungskatalysator verwendet wird.

6. Verfahren nach einem der Ansprüche 1—5, dadurch gekennzeichnet, daß die reduktive Aminierung bei einer Temperatur von 100—250°C durchgeführt wird.

7. Verfahren nach einem der Ansprüche 1—6, dadurch gekennzeichnet, daß je Mol des umzuwandelnden Ausgangsproduktes 1—3 Mol Ammoniak oder primäres Amin verwendet werden.

**Revendications**

1. Procédé de préparation d'un lactame par amination réductrice d'un composé oxo-alcanecarboxylique en présence d'un catalyseur d'hydrogénation, caractérisé en ce qu'un ester d'acide oxo-alcanecarboxylique avec 2 ou 3 atomes de carbone entre l'atome de carbone du groupe carbonyle et l'atome de carbone du groupe carboxyle est soumis à une amination réductrice en phase liquide en absence de solvant.

2. Procédé selon la revendication 1, caractérisé en ce que, comme ester, on utilise l'ester méthylique, éthylique ou isopropylique.

3. Procédé selon la revendication 1 ou 2, caractérisé en ce que, pour 100 g de produit de départ, on utilise 0,1—10 g de catalyseur calculé sous forme de métal.

4. Procédé selon l'une quelconque des revendications 1 à 3, caractérisé en ce qu'on utilise un catalyseur d'hydrogénation contenant du palladium, du platine ou du nickel.

5. Procédé selon la revendication 4, caractérisé en ce qu'on utilise un catalyseur d'hydrogénation contenant du nickel.

6. Procédé selon l'une quelconque des revendications 1 à 5, caractérisé en ce que l'amination réductrice est réalisée à une température de 100—250°C.

7. Procédé selon l'une quelconque des revendications 1 à 6, caractérisé en ce que par mole de produit de départ à convertir, on utilise 1—3 moles d'ammoniac ou d'amine primaire.